# EUROPEAN PATENT APPLICATION

(11) **EP 1 366 758 A1**
(43) Date of publication of application: **03.12.2003**
(21) Application number: 02711483.4
(22) Date of filing: 12.02.2002
(51) Int. Cl.: A61K 9/10, A61K 9/14, A61K 45/08, A61P 27/02

(54) **NOVEL OPHTHALMIC COMPOSITIONS**

(30) Priority: 15.02.2001 JP 2001038555
(71) Applicant: Sanwa Kagaku Kenkyusho Co., Ltd., Nagoya-shi, Aichi 461-8631 (JP)
(72) Inventor: KOUZAKI, Toshiyuki, c/o SANWA KAGAKU KENK. Co. Ltd, Nagoya-shi, Aichi 461-1860 (JP); NAGAO, Yoshiyuki, A105, NISHIKAISEI Sukaitaun, Yonago-shi, Tottori 683-0853 (JP)
(74) Representative: Lippert, Hans, Dipl.-Ing.
(86) International application number: JP0201170
(87) International publication number: WO02064114

(57) **Abstract**

The present invention is intended to provide an ophthalmic solution which can readily be administered and can comfortably be used without any pain in patients similarly to conventional ophthalmic solutions, and which can remain in the eyes for a long time whereby enabling sustained release of drugs similarly to ophthalmic ointments.

Thus, the present invention is to provide an ophthalmic composition consisting essentially of carrier particles comprising a carrier component and one or more kinds of active substance(s) for ophthalmic therapy dispersed or dissolved in this carrier component, wherein the specific gravity of said carrier particles is adjusted to not less than 1.2. The ophthalmic composition of the present invention is an aggregation of microparticles, and the specific gravity of the drug-containing carrier particles is preferably adjusted to not less than 1.2. It is used mainly in the form of ophthalmic solution containing the ophthalmic composition suspended in a dispersion medium

## Description

### TECHNICAL FIELD

The present invention relates to an ophthalmic composition. More particularly, the present invention relates to an ophthalmic composition which does not irritate or impair the conjunctiva or cornea when instilled into the eyes, and allows carrier particles containing an active substance for ophthalmic therapy to remain in the eyes for a long time, whereby enabling sustained release of said active substance for ophthalmic therapy for long-acting of drug efficacy.

### BACKGROUND OF THE INVENTION

In the field of ophthalmology, drugs are administered, for example, via instillation of ophthalmic solutions, application of ophthalmic ointments or use of injections. A method which is most convenient and gives no pain to patients is instillation of drug solutions. However, elimination of conventional ophthalmic solutions from the eyes is rapid, and its bioavailability is extremely low. Accordingly, frequent administration is required in many cases. As a result, patient compliance becomes to be low, and unnecessary administrations of drugs cause adverse reactions (M.TAKASUGI, Medicine and Drug Journal, 35 (9), 65, 1999).

For the above problems, dosage forms such as ophthalmic ointments, ophthalmic gels, ocular implants are being investigated. Though these formulations are superior to ophthalmic solutions in sustained efficacy, problems such as intraocular feelings of foreign body or stickiness remain unsolved. Especially, for ophthalmic ointments, difficulty in administration and discomfortable feeling in the eyes were pointed out (K.MOTOSE, TENGANZAI, NANZANDO, p.145-, 1986, S.IGUCHI, SHIN SOGO YAKUZAIGAKU <II>, ISHIYAKU SHUPPAN, p.146, 1981), and for ophthalmic gels recently developed, misty vision and discomfortable feeling are also pointed out.

On the other hand, ophthalmic suspensions containing drugs, etc. in instillable solvent are extensively examined, and J. W. Sieg *et al* reported that ophthalmic suspension containing suspension particles (polystyrene latex) with a particle size of 25 µm could be well retained in the anterior ocular segment (J.W.Sieg, J.Pharm. Sci., 69, 863, 1980). However, the retention time was up to 10 minutes after administration, and no long-time retention of drugs was observed in our studies. Accordingly, controlling of retention of drugs by polystyrene microparticles was difficult.

As mentioned above, among currently available ophthalmic solutions, drugs solutions including ophthalmic suspension have unsolved problems of short duration of drug efficacy and low bioavailability, and ophthalmic ointments, etc. have unsolved problems of inconvenient and uncomfortable administration. Accordingly, we made effort for achieving an objective which is to provide an ophthalmic solution which can easily be administered and can be used without pain in patients as conventional ophthalmic solutions, and which can remain in the eyes, whereby enabling sustained release of drugs as ophthalmic ointments.

### SUMMARY OF THE INVENTION

As a result, we discovered that an ophthalmic suspension containing polystyrene particles alone with a specific gravity of 1.05 could not be retained in the eyes, while by adjusting the specific gravity of carrier particles, delayed elimination of drugs from the eye can be achieved, whereby completing the present invention. That is, the present invention is to provide an ophthalmic composition consisting essentially of carrier particles containing a carrier component and one or more kinds of active substance(s) for ophthalmic therapy (hereinafter sometimes referred to as "drug" in this specification) dispersed or dissolved in this carrier component, in which the specific gravity of the carrier particles is adjusted, and is used mainly in the form that the ophthalmic composition is suspended in a dispersion medium. By adjusting the specific gravity of drug-containing carrier particles employed here to be not less than 1.2, delayed elimination of the drug-containing carrier microparticles from the eyes and maintaining the effective concentration of drugs in the eyes for a long time can be successfully achieved.

By controlling the specific gravity of carrier particles, the ophthalmic composition according to the present invention can provide improved intraocular retention of the preparation itself, i.e., a particulate carrier, and also enables sustained release of the active component from the particulate carrier, resulting in sustained efficacy of drugs. As a result, decreased frequency of instillation can lead to improvement of patients QOL, and reduced drug dose can lead to elimination of systemic adverse reactions, and therefore it is an extremely effective formulation in the field of ophthalmology.

### BRIEF DESCRIPTION OF THE DRAWINGS

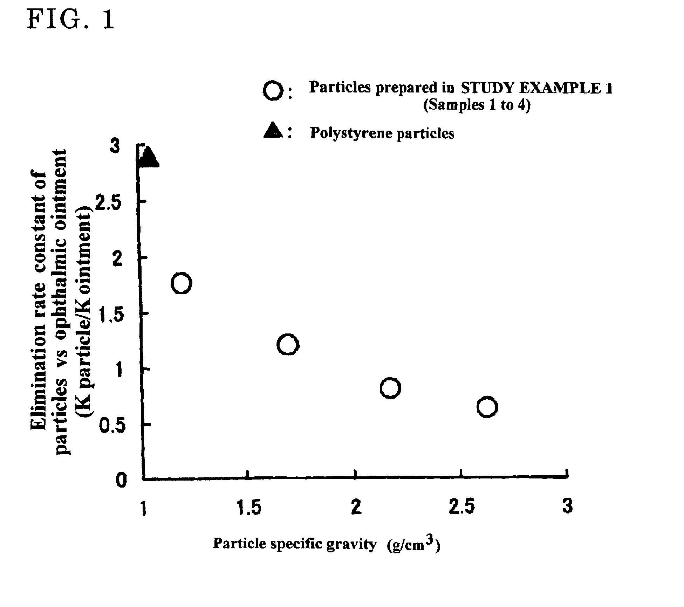

FIG. 1 represents, using the elimination rate of ophthalmic ointment as an index, the intraocular retention performance of carrier particles in STUDY EXAMPLE 1 with the specific gravity adjusted, by the relationship of elimination rate between the particle specific gravity and ophthalmic ointment. Each graph represents the mean value, and Sample 1 and Sample 4 were studied in 8 animals, and Sample 2 and Sample 3 in 6 animals.

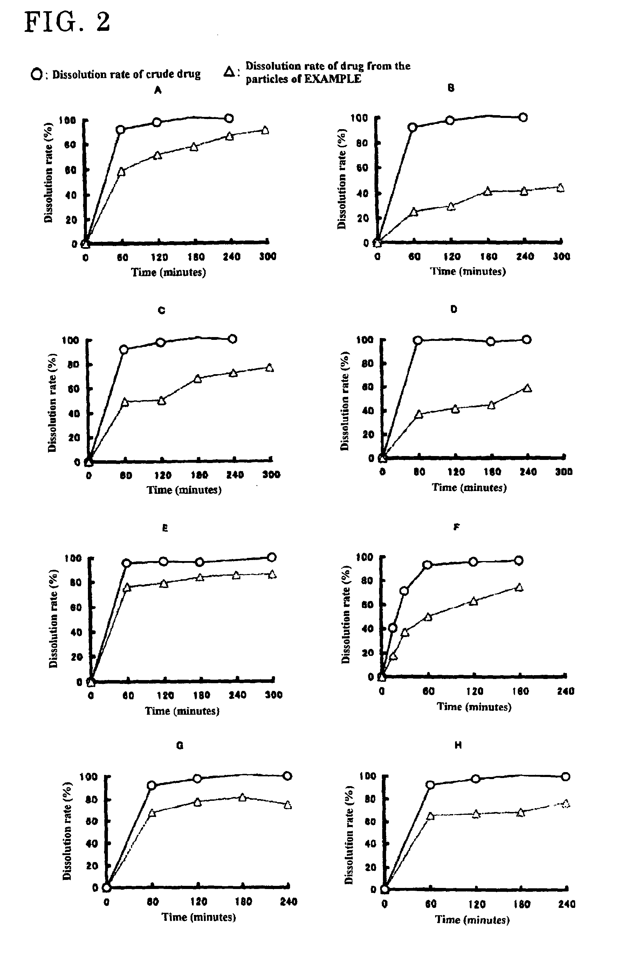

FIG. 2 represents the results of dissolution profiles of the particulate composition according to the present invention obtained in EXAMPLES 1-8 of Study Example 2. A to H corresponds to EXAMPLES 1 to 8, respectively.

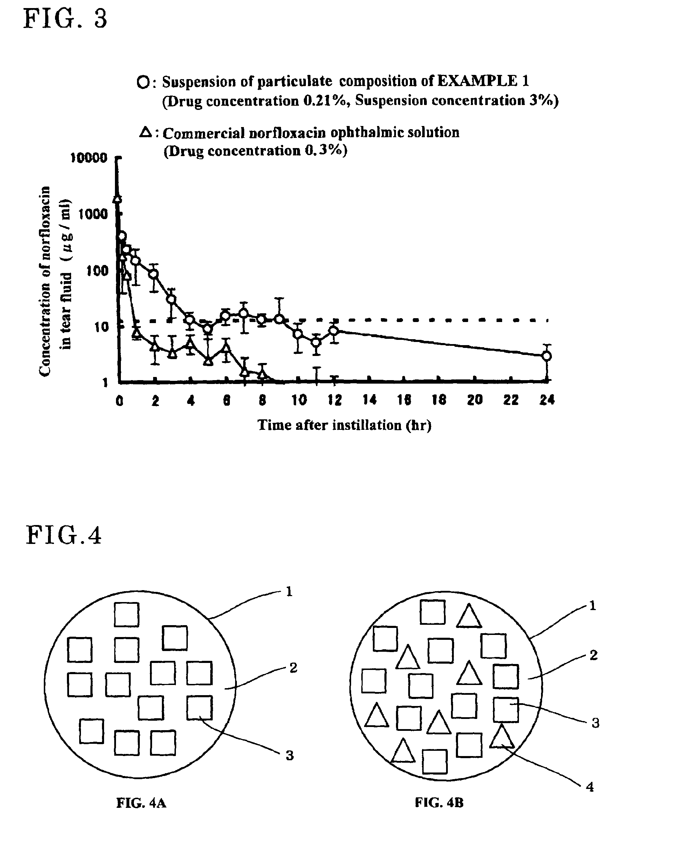

FIG. 3 represents the time-course change of the norfloxacin concentration in the tear fluid of a rabbit instilled ophthalmic suspension containing the particulate composition of EXAMPLE 1 of the present invention in comparison with an aqueous solution of norfloxacin as a control. Each graph is expressed in the mean ± standard deviation (n=4).
FIG. 4 represents a schematic view of a carrier particle. FIG. 4A represents a carrier particle 1 containing a carrier component 2 and an active substance 3 for ophthalmic therapy dispersed or dissolved in the carrier component 2. FIG. 4B represents a carrier particle 1 containing a carrier component 2, an active substance 3 for ophthalmic therapy and a specific gravity modifier 4.

### BEST MODE FOR CARRYING OUT THE INVENTION

The present invention is to provide an ophthalmic composition consisting essentially of carrier particles containing a carrier component and one or more kinds of active substance(s) for ophthalmic therapy dispersed or dissolved in this carrier component, in which the specific gravity of the carrier particles is adjusted. Said ophthalmic composition is an aggregation of a large number of microparticles (carrier particles), and may contain other components and the like than the carrier particles. The specific gravity of drug-containing carrier particles may be adjusted by adding a specific gravity modifier, or by selecting a carrier component having an appropriate specific gravity without using any specific gravity modifier. The specific gravity of carrier particles is usually adjusted to be not less than 1, preferably adjusted to be not less than 1.2, more preferably not less than 1.7, and most preferably to be not less than 2.0 for attaining excellent retention of the carrier particles. As used herein, specific gravity value means the specific gravity of carrier particles containing all of the carrier component and the drug as well as the specific gravity modifier added as necessary.

An active substance for ophthalmic therapy employed in the present invention may for example be a chemotherapeutic agent, antibiotic, anti-allergic agent, antiinflammatory agent, miotic agent, vitamin, vasoconstrictor, anti-histaminic agent, mydriatic agent, therapeutic drugs for glaucoma and cataract, local anesthetic, diagnostic agent for ophthalmic treatment, immunosuppressant, antimetabolite, decongestant, autonomic agent, therapeutic drug for diabetic retinopathy, amino acids and the like. It is a matter of course that any two or more of those listed above may be used in combination.

In the present invention, a carrier component containing active substance for ophthalmic therapy described above and controlling release of these substances may have description suitable for being formulated into a particle, and may be any component as long as it is physiologically acceptable. A carrier component having such characteristics may for example be a high molecular component, fatty component and the like, and those being preferred are listed below.

A non-water-soluble high molecular component as a carrier component may for example be ethyl cellulose, ethylene vinyl acetate copolymer, polymethyl methacrylate, ethyl acrylate - methyl methacrylate - trimethylammonium ethyl chloride methacrylate copolymer, methyl methacrylate - butyl methacrylate - dimethylaminoethyl methacrylate copolymer and the like A biodegradable high molecular component may for example be a polylactic acid, polylactic acid - glycolic acid copolymer, polycyanoacrylate, polyalkyl cyanoacrylate, poly-ε-caprolactone and the like. A water-soluble high molecular material may for example be a cellulose derivative such as hydroxypropylmethyl cellulose phthalate, carboxymethylethyl cellulose, hydroxypropyl cellulose and the like, as well as calcium alginate, chitosan, albumin, gelatin, methacrylic acid - methyl methacrylate copolymer and the like. A fatty component may for example be tripalmitine, cetyl alcohol, cholesterol, various phospholipids, cetyl palmitate, cholesterol palmitate and the like.

These carrier components usually have an ability of sustained-releasing an active component containing an active substance for ophthalmic therapy. Nevertheless, the carrier component employed in the present invention is not limited to those having such sustained-release function. That is, even the carrier particles of the present invention consisting of a carrier component having no sustained release function can retain an active ingredient in the eyes for a long time, during when the drug dispersed or dissolved in the carrier particles can be delivered in the eyes depending on the dissolution rate of the drug.

A carrier component with a high specific gravity usable without addition of any specific gravity modifier may for example be hydroxypropyl methyl cellulose phthalate 200731 (specific gravity: 1.65), hydroxypropyl methyl cellulose phthalate 220824 (specific gravity: 1.82), carboxymehtyl ethyl cellulose (specific gravity: 1.59) and the like. Nevertheless, even when such a carrier component is used, it is preferable to add a specific gravity modifier to further increase the specific gravity.

In the present invention, a specific gravity modifier employed for adjusting the specific gravity of carrier particles may for example be, but is not limited to, an insoluble component such as titanium oxide (specific gravity: 4.17), a hardly soluble component such as tricalcium phosphate (specific gravity: 3.14), anhydrous calcium hydrogen phosphate (specific gravity: 2.89), calcium hydrogen phosphate dihydrate (specific gravity: 2.30) and the like, a water-soluble component such as sodium chloride (specific gravity: 2.17), potassium chloride (specific gravity: 1.98), calcium chloride (specific gravity: 2.0), magnesium chloride (specific gravity: 2.41), sodium carbonate (specific gravity: 2.53), sodium dihydrogen phosphate (specific gravity: 1.95), sodium monohydrogen phosphate (specific gravity: 1.7), potassium dihydrogen phosphate (specific gravity: 2.34) and the like. Any of these specific gravity modifiers can be used as being contained in said carrier particles. Since each of these specific gravity modifiers has a different solubility or specific gravity, any of them can be employed alone or in combination with each other in an appropriate ratio, whereby enabling adjustment of the intraocular retention time of a preparation by varying the specific gravity. The specific gravity of the resultant carrier particles may vary depending on the type of the employed carrier component, type of the specific gravity modifier, type and amount of the active substance for ophthalmic therapy and the like.

For concrete method for use of specific gravity modifiers, a specific gravity modifier is selected which is not dissolved or sparingly dissolved in a dispersion medium (detailed below) employed for the ophthalmic composition of the present invention which is used as ophthalmic suspension. For example, when an aqueous dispersion medium is used for suspending carrier particles, it is preferable to use an insoluble component such as titanium oxide or a hardly soluble component described above. As a specific gravity modifier, one having a specific gravity far higher than the target specific gravity value of the carrier particles is selected preferably. A specific gravity modifier having a low specific gravity leads to an increased percentage of the specific gravity modifier in the carrier particles for obtaining intended specific gravity value of the carrier particles, resulting in reduced percentage of the carrier component to be added, which result in difficulty in exerting functions as carrier component, for example, the sustained-release function when the sustained-release function is expected.

From such a point of view, titanium oxide is preferred for preparing an ophthalmic composition having a specific gravity of not less than 2.0, since it is insoluble in various dispersion medium and has a specific gravity as high as 4.17. The specific gravity value of the resultant ophthalmic composition can be measured, for example, by a method specified in Japanese Pharmacopoeia, the Determination of Specific Gravity, Method 1. Measurement using a pycnometer.

The mean particle size of the ophthalmic composition according to the present invention, i.e., the mean particle size of carrier particles is preferably not larger than 75 µm usually, and more preferably not larger than 40 µm for the purpose of ameliorating intraocular uncomfortable feeling (According to J.P., Ophthalmic Solution, the particle size observed in suspensions for ophthalmic solutions is not larger than 75 µm). The particle size of the carrier particles of the present invention can be measured by the Sedimentation Method.

Particles with a particle size of not larger than several µm, generally not larger than 1 µm, is classified as colloid which is less affected by the specific gravity, and cannot provide sufficient intraocular retention. Accordingly, it is not preferable in the ophthalmic composition of the present invention to increase the percentage of microparticles with a particle size of not larger than 1 µm. Nevertheless, mixing said microparticles with a particle size of not larger than 1 µm can be expected to provide immediate-release effect of drugs from the microparticles and, therefore, a preparation having immediate- and sustained-release effects (by larger particle-size portion) can be realized.

It is preferable to administer the ophthalmic composition of the present invention intraocularly as ophthalmic suspension. When administering the composition as ophthalmic suspension, the particulate ophthalmic composition of the present invention is dispersed in a dispersion medium before use, and instilled similarly to ordinary ophthalmic solutions. It is also possible to prepare an ophthalmic suspension previously.

When the ophthalmic composition of the present invention is used as ophthalmic suspension, aqueous or non-aqueous dispersion mediums selected on the basis of the solubility of the active substance for ophthalmic therapy and carrier component can be employed. For aqueous dispersion medium, when calcium alginate is used as a carrier component, aqueous solution of calcium chloride in which the carrier particles are insoluble may be used as dispersion medium, or saturated aqueous solution of drugs which prevents outflowing drugs from carrier particles may be used.

For non-aqueous dispersion medium, when water-soluble carrier component is used, dispersion mediums in which the water-soluble carrier component and the active substance for ophthalmic therapy are insoluble may be used, such as vegetable oils including an olive oil, sesame oil, soybean oil, camellia oil, rapeseed oil, corn oil, peanut oil, cottonseed oil, and liquid paraffin, propylene glycol, glycerin, β-octyl dodecanol and the like. Ophthalmic suspension may be added with additives usually used for manufacturing of ophthalmic drugs, such as an excipient, suspending agent, pH modifier, buffer, osmotic agent, preservative, thickening agent and the like. Any of such additives may previously be contained in the ophthalmic composition of the present invention.

The composition of the present invention can be prepared by any method. Several preparation methods are exemplified below.
1. A carrier component is dissolved in an appropriate solvent, an active substance for ophthalmic therapy is dissolved or dispersed in the mixture, and a specific gravity modifier is dispersed, after that the mixture is formed to a film solid by the casting method, and pulverized to obtain carrier particles.
2. A carrier component is dissolved in an appropriate solvent, an active substance for ophthalmic therapy is dissolved or dispersed in the mixture, and the dispersion is formed to carrier particles by the spray drying method.
3. A carrier component is dissolved in a solvent, an active substance for ophthalmic therapy (drug) or a specific gravity modifier is dissolved or dispersed in the mixture, and the surface of core particles is coated by the wuster coating to obtain carrier particles (among the specific gravity modifier and the drug, one which has not been dissolved or dispersed first in the solvent serves as a core).
4. A carrier component is dissolved in a solvent and core particles obtained by granulating an active substance for ophthalmic therapy and a specific gravity modifier by small amount of the carrier component are dispersed, and non-solvent is added to form carrier particles by the coacervation method to form a carrier component film on the core surface.
5. A carrier component is dissolved in a solvent, and an active substance for ophthalmic therapy is further dissolved. To this, a specific gravity modifier is dispersed as core particles, and then the other solvent in which the carrier component is insoluble added to form carrier particles by the coacervation method to form a carrier component film on the surface of the core particles.

Carrier particles having a desired particle size can readily be obtained by sieving the carrier particles obtained. It is also possible to verify the carrier particle size by measuring with the centrifugal sedimentation particle size distribution meter, etc.

### EXAMPLES

### EXAMPLE 1 (Norfloxacin-containing ethyl cellulose particle)

Ethyl cellulose (633 mg) was dissolved in a mixture of ethanol-dichloromethane (1:1) (10 ml), to which norfloxacin (150 mg) was added and dissolved, and titanium oxide (2.22 g) was further dispersed to the mixture. The mixture was formed to a sheet by the casting method. The sheet was pulverized and sieved (20 to 32 µm) to obtain carrier particles with a specific gravity of 2.42.

### EXAMPLE 2 (Norfloxacin-containing polycyanoacrylate particle)

Polycyanoacrylate (570 mg) was dissolved in dichloromethane (10 ml), to which norfloxacin (154 mg) was added and dissolved, and titanium oxide (2.28 g) was further dispersed to the mixture. The mixture was formed to a sheet by the casting method. The sheet was pulverized and sieved (20 to 32 µm) to obtain carrier particles with a specific gravity of 2.40

### EXAMPLE 3 (Norfloxacin-containing cetyl palmitate particle)

Cetyl palmitate (570 mg) was dissolved in dichloromethane (10 ml), to which norfloxacin (154 mg) was added and dissolved, and titanium oxide (2.28 g) was further dispersed to the mixture. The mixture was formed to a sheet by the casting method. The sheet was pulverized and sieved (20 to 32 µm) to obtain carrier particles with a specific gravity of 2.46.

### EXAMPLE 4 (Pranoprofen-containing ethyl cellulose particle)

Ethyl cellulose (633 mg) was dissolved in ethanol (10 ml), to which pranoprofen (150 mg) was added and dissolved, and titanium oxide (2.22 g) was further dispersed to the mixture. The mixture was formed to a sheet by the casting method. The sheet was pulverized and sieved to obtain carrier particles with a particle size of 20 - 32 µm whereby obtaining carrier particles with a specific gravity of 2.41.

### EXAMPLE 5 (Tranilast-containing cethyl palmitate particle)

Cethyl palmitate (570 mg) was dissolved in dichloromethane (10 ml), to which tranilast (154 mg) was added and dissolved, and titanium oxide (2.28 g) was further dispersed to the mixture. The mixture was formed to a sheet by the casting method. The sheet was pulverized and sieved to obtain carrier particles with a particle size of 20 - 32 µm whereby obtaining carrier particles with a specific gravity of 2.14.

### EXAMPLE 6 (Fluorometholone-containing ethyl cellulose particle)

Ethyl cellulose (633 mg) was dissolved in ethanol (10 ml), to which fluorometholone (150 mg) was added and dissolved, and titanium oxide (2.22 g) was further dispersed to the mixture. The mixture was formed to a sheet by the casting method. The sheet was pulverized and sieved to obtain carrier particles with a particle size of 20 - 32 µm whereby obtaining carrier particles with a specific gravity of 2.46.

### EXAMPLE 7 (Norfloxacin-containing cethyl palmitate particle)

Cethyl palmitate (851 mg) was dissolved in dichloromethane (10 ml), to which norfloxacin (242 mg) was added and dissolved, and titanium oxide (1.91 g) was further dispersed to the mixture. The mixture was formed to a sheet by the casting method. The sheet was pulverized and sieved to obtain carrier particles with a particle size of 20 - 32 µm whereby obtaining carrier particles with a specific gravity of 1.76.

### EXAMPLE 8 (Norfloxacin-containing cethyl palmitate particle)

Cethyl palmitate (1.55 mg) was dissolved in dichloromethane (10 ml), to which norfloxacin (290 mg) was added and dissolved, and titanium oxide (1.17 g) was further dispersed to the mixture. The mixture was formed to a sheet by the casting method. The sheet was pulverized and sieved to obtain carrier particles with a particle size of 20 - 32 µm whereby obtaining carrier particles with a specific gravity of 1.20.

### STUDY EXAMPLES

As used herein, concentration (%) means weight/volume (%) unless otherwise specified. % by weight as specified means weight/total weight (%).

### STUDY EXAMPLE 1

### Evaluation of intraocular retention of ethyl cellulose particles in rabbits

### [Method]

Ethyl cellulose (260 mg) was dissolved in chloroform (6 ml). Titanium oxide (1.904 g) and FD&C Yellow No.4 aluminum lake (835 mg) were added to this solution and stirred. The aggregation of titanium oxide and the like was pulverized ultrasonically to disperse as far as possible. This mixed suspension was cast onto teflon petri dish, and allowed to dry naturally. The film thus obtained was pulverized and sieved (20 to 32 µm) to obtain intended particles (Table 1, Sample 1). By varying the composition ratio of each component and using similar method, carrier particles having various specific gravity were prepared (Table 1, Samples 2, 3 and 4). The carrier particle size was measured by the centrifugal sedimentation particle size distribution meter for confirmation. Table 1 represents the median size.

The carrier particles thus prepared were suspended in isotonic phosphate buffer (pH7.4) at 2.6%, and fifty microliters of the suspension was instilled into the lower conjunctival sac of a rabbit. After a certain time has passed, the particles in the eyes were recovered, and the % residue of the particles was measured using a dye as an index. As a control, ophthalmic ointment containing FD&C Yellow No.4 aluminum lake (19.8 mg) in plastibase (7.40 g) was prepared, and the ointment prepared was applied onto the lower conjunctival sac of a rabbit. After certain time has passed, the ophthalmic ointment in the eyes was recovered, and the % residue of the ophthalmic ointment was measured using a dye as an index. The elimination rate was compared between the carrier particles with different specific gravity assuming the elimination rate constant of the ophthalmic ointment as 1. As a reference, polystyrene particles (Fluoresbrite™ Plain Microspheres; 2.61% Solid-Latex, Polysciences, Inc.) were subjected to the same test for confirmation of the current technique. The % residue of this particle was determined by recovering the particles from the eyes, similarly to the samples, and using as an index the fluorescent intensity of the fluoresceine.

**Table 1**

| | Sample 1 | Sample 2 | Sample 3 | Sample 4 | Reference Standard |
|---|---|---|---|---|---|
| Specific gravity | 2.63 | 2.17 | 1.70 | 1.20 | 1.05 |
| Median Diam. (µm) | 26.85 | 28.15 | 29.32 2 | 31.09 | 25.0 |
| | | | | | |
| Composition [% by weight] | | | | | |
| FD&C Yellow No.4 aluminum lake | 13.86 | 27.96 | 28.11 | 15.14 | Fluoresceine |
| Titanium oxide | 71.04 | 54.62 | 42.70 | - | |
| Ethyl cellulose | 15.11 | 17.42 | 29.19 | 84.86 | Polystyrene |

### [Results]

The results of the experiment are shown in FIG. 1. Polystyrene particles with a specific gravity of 1.05 was rapidly eliminated from the eyes, while the ethyl cellulose particles whose specific gravity had been adjusted exhibited an improved intraocular retention. Especially, when the specific gravity was adjusted to not less than 2, intraocular retention became longer than that achieved by ophthalmic ointments which is believed to have excellent intraocular retention.

### STUDY EXAMPLE 2

### Evaluation of performance of drug-containing particles with the specific gravity adjusted

### [Method]

Each of the drug-containing particles (10 to 20 mg) prepared in EXAMPLES 1 to 8 was immersed in pH7.4 isotonic phosphate buffer (40 ml), the dissolution amount of drugs from the particles was measured under the study conditions of 37°C and 100 rpm with spectrophotometer or liquid chromatography, and the dissolution rate was calculated from the drug dose at each time interval. As a control, for each of the drugs contained in the particles of Examples, the dissolution rate of the drugs from the crude powder was calculated in the similar manner. In the control group, the study was performed by adjusting the added amount of each drug (crude powder) so as the concentration in the test solution to be below the saturated solubility of the drug.

### [Results]

The results obtained are shown in FIG. 2 A to H. As evident from the figure, the sustained release property of the active ingredient was verified in the ophthalmic composition of the present invention.

### STUDY EXAMPLE 3

Changes in the norfloxacin concentration in the tear fluid of rabbits after administration of norfloxacin-containing ethyl cellulose composition

### [Method]

The particles prepared in EXAMPLE 1 were suspended at 3% [corresponding to 0.15% as norfloxacin concentration] in a saturated aqueous solution (0.06%) of norfloxacin to prepare suspension containing 0.21% norfloxacin as a whole. This suspension (50 µl) was instilled into the lower conjunctival sac, and the concentration of norfloxacin in the anterior ocular segment was measured at certain time interval with liquid chromatography. As a control, commercial 0.3% norfloxacin ophthalmic solution (NOFLO ophthalmic solutions) was instilled similarly.

### [Results]

The results are shown in FIG. 3. The MIC₈₀ values (12.5 µg/ml) of norfloxacin against *Streptococcus sp* which as a clinical isolate of the ocular infections was employed as an index of the effective concentration. The duration of the effective concentration obtained from the results shown in FIG. 3 was presented in Table 2. The duration of the effective concentration of norfloxacin in the inventive formulation-treated group employing suspension particles was 9 times longer than that in the control group treated with the commercial ophthalmic solution in which the drug content was 1.5 times higher.

**Table 2**

| | EXAMPLE 1, Ophthalmic suspension | Norfloxacin aqueous solution |
|---|---|---|
| MIC₈₀ retention time in the tear fluid against *Streptococcus sp.* | 9 hours | 1 hour |

### STUDY EXAMPLE 4

### Safety evaluation of particle treatment

### [Method]

Particles with a particle size of 25 µm and specific gravity of 2.6 consisting of ethyl cellulose (30.4 % by weight) and titanium oxide (69.6 % by weight) was prepared, and suspended at 3.0% in isotonic phosphate buffer (pH7.4). The suspension was instilled into the lower conjunctival sac of a rabbit twice daily continuously for 28 days, and irritation and damage during instillation and after completion of instillation were assessed by Draize method, fluorescein staining using physiological saline as control.

### [Results]

As shown in Tables 3 and 4, no irritation or damage was noted by the instillation of carrier particles.

**Table 3**

| Ocular mucosa irritation in rabbits | | | | | | |
|---|---|---|---|---|---|---|
| Sample | Tissue site | Day of evaluation | | | | |
| | | Day 1 of administration (n=5) | Day 7 of administration (n=5) | Day 14 of administration (n=5) | Day 21 of administration (n=5) | Day 28 of administration (n=5) |
| Microparticle suspension | Cornea | 0 | 0 | 0 | 0 | 0 |
| | Iris | 0 | 0 | 0 | 0 | 0 |
| | Conjunctiva | 0 | 0 | 0 | 0 | 0 |
| | Total | 0 | 0 | 0 | 0 | 0 |
| Physiological saline | Cornea | 0 | 0 | 0 | 0 | 0 |
| | Iris | 0 | 0 | 0 | 0 | 0 |
| | Conjunctiva | 0 | 0 | 0 | 0 | 0 |
| | Total | 0 | 0 | 0 | 0 | 0 |

**Table 4**

| Fluorescein staining of rabbit cornea | | | |
|---|---|---|---|
| Sample | Percentage of cornea identified fluorescein stain (%) | | |
| | Before start of the study (before 4 weeks) | 12 hours after final instillation | 24 hours after final instillation |
| Microparticle suspension | 0 (0/5) | 0 (0/5) | 0 (0/5) |
| Physiological saline | 0 (0/5) | 0 (0/5) | 0 (0/5) |

After the final instillation, each rabbit was immobilized on a yoke fixer for the purpose of avoiding any contact of extremities with the region around the eyes.

## Claims

1. An ophthalmic composition consisting essentially of carrier particles comprising a carrier component and one or more kinds of active substance(s) for ophthalmic therapy dispersed or dissolved in the carrier component, wherein the specific gravity of said carrier particles is adjusted to not less than 1.2.

2. The ophthalmic composition according to Claim 1, wherein the specific gravity of said carrier particles is adjusted by a specific gravity modifier.

3. The ophthalmic composition according to Claim 1 or 2, wherein said carrier component is a high molecular carrier.

4. The ophthalmic composition according to Claim 1 or 2, wherein said carrier component is a fatty carrier.

5. The ophthalmic composition according to any one of Claims 1 to 4, wherein the particle size of said carrier particles is not larger than 75 µm.

6. A ophthalmic solution containing the ophthalmic composition according to any one of Claims 1 to 5 which is suspended in a dispersion medium.
